# EUROPEAN PATENT APPLICATION

(11) **EP 4 485 938 A1**
(43) Date of publication of application: **01.01.2025**
(21) Application number: 23182880.7
(22) Date of filing: 30.06.2023
(51) Int. Cl.: H04N 21/214, G16H 80/00, H04N 21/231, H04N 21/234, H04N 21/2343, H04N 21/236, H04N 21/2381, H04N 21/2385, H04N 21/262, H04N 21/2662, H04N 21/845

(54) **VIDEO ENCODER AND RELATED METHODS**

(71) Applicant: Baxter Medical Systems GmbH + Co. KG, 07318 Saalfeld (DE)
(72) Inventor: SALVATERRA, Davide, 07318 Saalfeld (DE); CONTESSI, Paolo, 07318 Saalfeld (DE); BASSO, Andrea, 07318 Saalfeld (DE); FURGONI, Stefano, 07318 Saalfeld (DE); PONTISSO, Stefano, 07318 Saalfeld (DE); MORASSUTTI, Aurelio Carlos, 07318 Saalfeld (DE)
(74) Representative: Reddie & Grose LLP

(57) **Abstract**

A method of encoding an operating room video feed into a video over internet protocol (VoIP) stream is provided. The method comprises receiving a video feed; splitting the video feed into a first video stream and a second video stream; encoding the first video stream into a first VoIP feed; and encoding the second video stream into a second VoIP feed. The second video stream is compressed when it is encoded such that the second VoIP feed has a lower bit rate than the first VoIP feed. A corresponding encoder, system and non-transitory computer readable memory are also provided.

## Description

### TECHNICAL FIELD

The present invention relates to methods, devices and systems for managing video feeds. In particular, the present invention relates to methods of encoding an operating room video feed over a video over internet protocol stream and a related video encoder, system and non-transitory computer readable memory.

### BACKGROUND

Operating rooms of healthcare facilities typically have one or more video cameras to capture video of procedures for patient care, educational, review, or other purposes. Typically, a video feed is captured by a camera, processed by equipment within an operating room into a video over internet protocol (VoIP) stream and sent over a network for storage, streaming, etc. Having each operating room working as a standalone system can introduce bottlenecks and limit the amount of channels that can be recorded in an operating room, as well as requiring a large amount of physical infrastructure in each operating room having both a space and monetary cost.

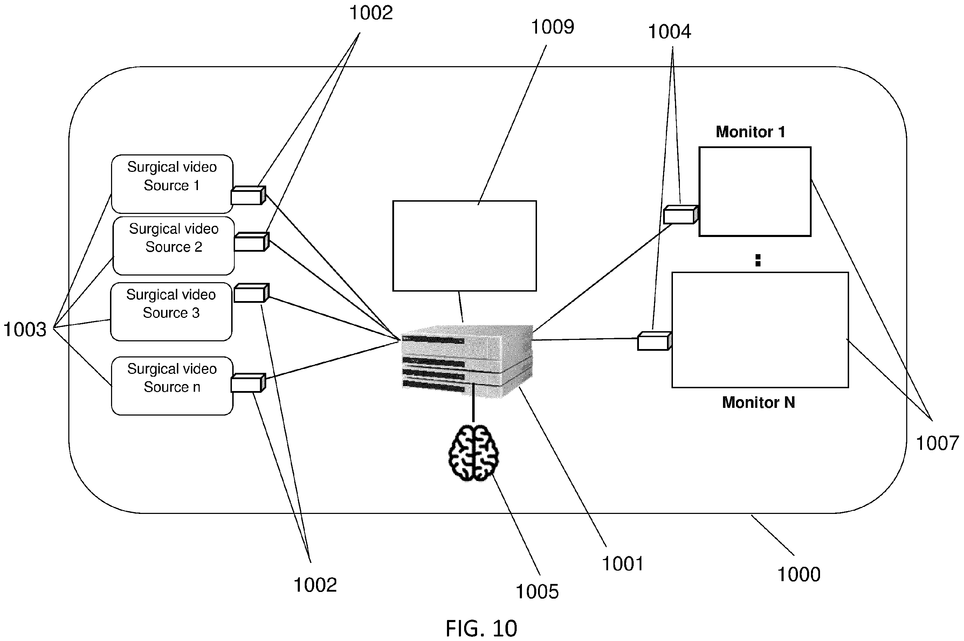

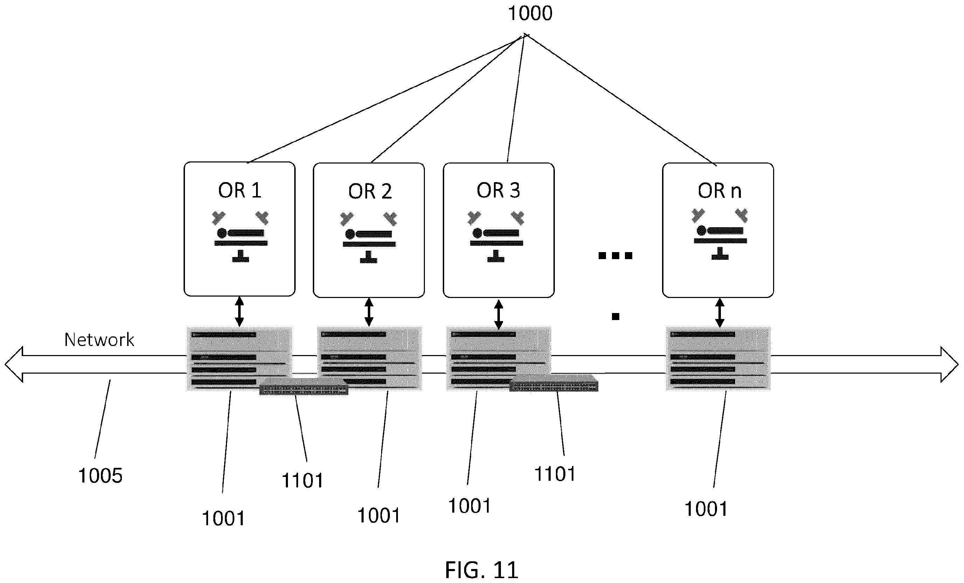

Figures 10 and 11 illustrate such arrangements according to the prior art.

Figure 10 (prior art) illustrates a system for obtaining medical data in an operating room 1000, and in particular video data or a video stream. Each individual operating room (OR) is connected to a hardware infrastructure belonging only to that specific OR. Devices 1003 including video data sources are all connected through hardware connections to a central unit 1001 configured to perform all necessary hardware and software requirements. The devices 1003 may be in connection with the central unit 1001 via encoders 1002 such as VoIP encoders. The central unit 1001 also comprises a routing matrix, with each routing matrix 1001 being in communication with a network 1005 such as a healthcare facility or hospital network.

The central unit 1001 outputs video data from the devices 1003 to be displayed on one or more monitors 1007 via fibre transceivers 1004, to be viewed in the OR.

The central unit 1001 is also in connection with a computing device 1009 for displaying a user interface configured to interact with one or more devices in the OR.

Figure 11 illustrates a plurality of ORs 1000 including ORs 1 to n, each having a corresponding central unit 1001 illustrated in Figure 10. Each corresponding central unit 1001 performs the necessary hardware and software requirements for each OR, and connects to the network 1005.

The capabilities of the central units 1001 may include VoIP functionality, but each OR still comprises its own hardware infrastructure. Two ORs may share a routing matrix 1101 for connecting with the network 1005.

### SUMMARY OF THE INVENTION

The invention is defined by the independent claims to which reference should now be made. Optional features are set forth in the dependent claims.

Inventors of the present disclosure have appreciated the need for a distributed intelligence system provided in a healthcare facility or hospital environment, for example across a plurality of ORs. Significantly, this removes the need for individual hardware systems, which may be referred to as "stacks" or "racks", corresponding to each OR.

By providing such a distributed intelligence system, embodiments of the present disclosure remove the need for each OR working as a standalone system. This advantageously removes corresponding bottlenecks, removes the limit of channels on which on which the system can record, removes the need for local storage of data such as video data and thus the requirement to export content to a centralized storage system for later access, removes the need for VoIP conferencing equipment to be present in every OR thereby reducing cost, and reduces the overall physical footprint as hardware stacks are not required in each OR.

The inventors of the present disclosure have also appreciated the need to be able to provide continuous recording within an OR without necessarily requiring a user trigger. Embodiments described herein therefore provide a device such as an encoder capable of recording video data without necessarily requiring a user trigger. The distributed intelligence system according to embodiments described herein may comprise such an encoder.

One aspect of the disclosure relates to a method of encoding an operating room video feed into a video over internet protocol (VoIP) stream. The method comprises: receiving a video feed; splitting the video feed into a first video stream and a second video stream; encoding the first video stream into a first VoIP feed; encoding the second video stream into a second VoIP feed. The second video stream is compressed when it is encoded such that the second VoIP feed has a lower bit rate than the first VoIP feed.

Splitting the video feed into a first video stream and a second video stream enables different encoding to be performed on the two video streams. The two video streams can, therefore, then be optimised for different uses, such as streaming and recording.

Optionally, the second video stream is compressed using one of HEVC, H.264, and AV1 compression.

Such relatively heavy compression codecs greatly reduce the bit rate of the second video feed allowing it to be transmitted over a network whilst placing much less burden on the network infrastructure.

Optionally, the first video stream is compressed when it is encoded into the first VoIP feed. In particular, optionally the first stream is encoded using a visually lossless compression.

Using visually lossless compression enables the first video feed to be optimised for uses where it is important not to lose detail in the video image and wherein the network infrastructure can support a higher bit rate.

Optionally, the first video stream is encoded such that the first VoIP feed has a bit rate of between 5 and 6 gigabits per second and/or wherein the second video stream is encoded such that the second VoIP feed has a bit rate of less than 0.2 gigabits per second.

Optionally, the method further comprises determining at least one property of the video feed. In this case, the encoding of the first video stream and/or the encoding of the second video stream may be based upon the determined at least one property of the video feed.

Determining a property of the video feed and basing the encoding of at least one of the first and second video streams on the determined property makes the method adaptable to different inputs and video sources, such as cameras with different resolutions or framerates.

Optionally, the at least one determined property of the video feed comprises a resolution of the video feed. In this case, the encoding of the first video stream and/or the encoding of the second video stream may be based upon the resolution of the video feed.

In this case, the encoding of the first video stream and/or the encoding of the second video stream comprises scaling the first video stream and/or scaling the second video stream based upon the resolution of the video feed.

Optionally, method further comprises receiving a resolution of a display. In this case, scaling the first video stream and/or scaling the second video stream is further based upon the resolution of the display.

Optionally, the method further comprises: outputting the first VoIP feed as a low latency stream; and outputting the second VoIP feed as an asynchronous stream.

Outputting the first VoIP stream as a low latency feed and the second VoIP feed as an asynchronous stream further increases the suitability of the first VoIP stream for real time uses and the second VoIP for (external) network uses.

Optionally, outputting the second VoIP feed as an asynchronous stream comprises: dividing the second VoIP feed into a plurality of fragments; and sending the plurality of fragments in an asynchronous manner over a network.

Optionally, prior to outputting the plurality of fragments in an asynchronous manner, one or more of the plurality of fragments are buffered.

Optionally, the plurality of fragments are output from an encoder or a switching device, and wherein the method further comprises: receiving, at a server, the plurality of fragments; and storing the plurality of fragments on the server. Optionally the method further comprises combining, by the server, the plurality of fragments into an ordered playlist of the fragments or to recreate the second VoIP feed.

Receiving the fragments of the second VoIP feed at a server and, in some cases, combining the plurality of fragments into an ordered playlist or to recreate the second VoIP feed allows for the second VoIP feed to be utilised for long term storage and streaming purposes, amongst other non-real time uses.

Another aspect of the disclosure relates to a video encoder or a system comprising a video encoder and a server configured to perform the method of the preceding aspect of the disclosure.

Another aspect of the disclosure relates to a non-transitory computer readable memory having stored thereon instructions that, when executed by a video encoder or system according to the second aspect of the disclosure, cause the video encoder or system to perform the method the first aspect of the disclosure above.

According to another aspect of the disclosure, there is provided a system for obtaining medical data in an operating room, the system comprising: an encoder having: an input configured to receive a video stream of medical data; a recording module; and a trigger module configured to, in response to a trigger command, trigger the recording module to begin recording the video stream; and a video stream source configured to provide the video stream to the encoder. A video stream of medical data may be any video stream related to real-time operations or procedures in the OR. This may include, but is not limited to, video streams related to cameras, surgical equipment, patient monitoring hardware, and conferencing systems such as VoIP systems within an OR. Such video sources may non-exhaustively include x-ray equipment, endoscopy equipment, room cameras, surgical cameras, monitoring equipment, a Hospital Information System (HIS), and a Picture Archiving and Communication System (PACS).

The encoder may comprise the functionality of encoders according to embodiments described herein.

The trigger command may comprise the encoder receiving a video feed. For example, the trigger module of the encoder may be configured to trigger the recording module to begin recording the received video stream when the encoder receives the video stream. That is, as soon as a video stream or video signal is detected by the encoder, recording may be started. In such an embodiment, no user interaction is necessary to begin recording. Advantageously, this prevents any user error related to a user not starting recording, or delaying recording, meaning no video data is inadvertently missed.

The trigger command may be set according to actions performed in the OR. For example, the trigger command may comprise an action occurring in the OR. This may be an interaction by a user with a device in the OR. The trigger command may comprise an input from a user, for example including a command to begin recording, or a command with an instruction as to when to begin recording.

Optionally, the encoder further comprises a local storage, the encoder being configured to buffer the video stream to the local storage. The encoder may therefore be configured to buffer the video stream to the local storage in response to the trigger command, such as the detection of a video stream or signal.

According to embodiments described herein, the encoder may provide the video stream to a server or network. By storing at least some of the video stream locally at the encoder, this prevents a server or network being flooded with unnecessary data. This also allows a system integrating the encoder to be more resilient to network conditions and preventing server overload.

Optionally, the system further comprises a device such as a media asset manager configured to receive at least some of the buffered video stream from the encoder.

Optionally, the encoder is configured to provide the at least some of the buffered video stream to the media asset manager when it is determined that the at least some of the buffered video stream is of interest. This may be after significant buffering, such as hours of local buffering at the encoder. In some embodiments, all of the locally buffered video data may be provided to the media asset manager. The encoders may be configured to record and synchronize the video streams for the real-time medical data.

Video data may be determined as being of interest following a user input or interaction, which may occur in the OR, at the media asset manager, or may occur centrally at the server side of a distributed intelligence system. In some embodiments, the media asset manager is configured to receive a user input indicating that the at least some of the buffered video stream is of interest. For example, a user may determine that video data recorded at a certain time, or by a certain device, is of interest, at which time this video data may be provided to the media asset manager. Again, this prevents the server from needing to process and store unnecessary video data.

In addition, the inventors of the present disclosure have appreciated that the current export process of video streams or video data is an unnecessary, time consuming step. By providing an encoder capable of recording a video stream in response to a trigger command, locally buffering at least some of the video stream, and providing at least some of the video stream to the media asset manager when it is determined that the data is of interest, the export process of the video stream is streamlined and more efficient.

In some embodiments, the media asset manager is part of the distributed intelligence system and is not separated.

According to another aspect of the present disclosure, there is provided an encoder for obtaining medical data in an operating room, the encoder comprising: an input configured to receive a video stream of medical data; a recording module; and a trigger module configured to, in response to a trigger command, trigger the recording module to begin recording the video stream.

According to another aspect of the present disclosure, there is provided a system for obtaining medical data in one or more operating rooms, the system comprising: a core module coupled to at least one encoder; a video stream source coupled to the at least one encoder, the at least one encoder being configured to receive and record a video stream of medical data from the video stream source; at least one decoder configured to receive the video stream from the encoder; and a server architecture in communication with the core module and being configured to receive at least some of the video stream from the at least one encoder via the core module, wherein the core module is configured to be coupled to one or more additional encoders and/or decoders.

The system for obtaining medical data provides the distributed intelligence described herein. Systems according to embodiments described herein allow scalability and provide the ability to expand existing installed systems in terms of capacity, for example the numbers of inputs and outputs. A "distributed" system may refer to a cloud based computing arrangement, which in some examples may be an on premises cloud based system. Cloud based resources are typically remotely separate from end-user systems and are shared by multiple users. With cloud computing, those multiple users (for example, relating to multiple ORs) can access a single server or group of servers, which may be distributed over a number of central processing units (CPUs), to retrieve, update, and process their data.

Broadly, in the described system, a core module may be provided in each OR of the one or more ORs. The core module facilitates connection between encoders and decoders as described herein, and a server architecture on a network infrastructure. The processing functionality and "intelligence" is provided by the encoders and decoders, and devices located at the server architecture side of the system. Each core module in corresponding ORs connects to the server architecture, and the storage and processing of video streams provided by devices in the OR is provided by the encoders, decoders, and on the server architecture side of the system. In other words, there is distributed intelligence or processing across the system covering a plurality of ORs, but centralized per operating room due to the functionality of the devices. Central processing may be provided through the server architecture, and to incorporate another OR into the network, a core module in connection with OR devices may be installed in an OR, connecting the OR to the server architecture.

The encoders comprise hardware units which may be configured to collect real-time medical data streams. The system may additionally comprise a control interface, which may be present in each OR, and be configured to interact with and control the encoders and decoders. The control interface may comprise a touch screen control system configured to receive user inputs for providing interaction with one or more devices in the OR. The control interface may also be in communication with a conferencing system provided by the server architecture. That is, a conferencing system may be shared among all ORs as the processing is provided through the server architecture, thereby removing the need for dedicated conferencing equipment to be provided within each OR.

Optionally, the core module comprises a switch or gateway including a plurality of ports configured to be coupled to the one or more additional encoders and/or decoders. The switch or gateway may allow transmission of video streams from the encoders to the network infrastructure. The core module may be configured to be coupled to a plurality of additional encoders and decoders. Conveniently, additional operating rooms may be incorporated into the system merely by including additional core modules connected to additional encoders and/or decoders. In addition, in each OR, additional encoders/decoders may be added to the system through coupling to the core module. This allows improvement of reliability and capacity of the system by adding the required respective components. This ultimately allows for convenient expansion of the system without requiring full hardware and software setups to be installed in each OR.

Optionally, the server architecture is configured to store the at least some of the video stream. The server architecture may store at least some of the video streams from each OR in the system, the server architecture being in communication with a core module associated with each OR. This centralized recording on the server architecture removes the need to export video data from each OR. In addition, encoders according to embodiments described herein allow for recording of multiple signals within a single OR (and across multiple ORs) as the number of channels on which recording can be performed is not limited.

The server architecture may comprise a first server or a fast server. The fast server may be physically located in the OR area and be in connection with each OR. This connection may be via a network infrastructure such as an ethernet network capable of providing a relatively fast 10Gbps bandwidth. The fast server may comprise the switch or gateway described herein, such as a 10Gbps switch. The fast server may comprise a Multiview processor. The fast server may provide the conferencing system functionality enabling videoconferencing in each OR without requiring dedicated conferencing equipment to be present in each OR. The fast server may also provide audio management for managing audio in relation to the video streams, as well as videoconferencing.

The server architecture may also comprise a heavy server. The heavy server may be a physical server implemented with hardware components or may be a virtualized server. The heavy server may be in connection with the fast server and the 10Gbps network via a network trunk. The heavy server may be in connection with a larger hospital network. The heavy server may be implemented at a data centre. The heavy server may provide additional services not required in real time. A user may access medical data in the form of video streams stored at the server or data centre and recorded in an OR by the encoders remotely via the network. This medical data may then be processed.

The system may further comprise a media asset manager or media asset management system, which may be provided at the heavy server. The media asset manager may be configured to store at least some of the video streams provided by one or more ORs.

The fast server and heavy server may together form the cloud based computing system. This may be configured to enable integration with one or more additional systems on a wider hospital network. For example, the cloud based computing system may non-exhaustively incorporate a real-time locating system (RTLS), a scheduling system, and a streaming platform.

The application of a local cluster of fast servers working together in a small local cloud provides support each OR. This thereby improves redundancy, resiliency, failover, and load balancing. For example, the fast server may be located relatively close to the ORs, thereby being close to OR devices as described. Storage, processing, and other services are then provided at the heavy server, for example at the data centre, that don't need to be located close to each OR.

Optionally, the core module is configured to connect to the server via a network. The connection may be a wired connection such as an ethernet connection, or may be a wireless connection such as a wireless local area network connection.

As described herein, the system may obtain medical data from a plurality of operating rooms. The core module may be a first core module which may be associated with a first OR. The system may therefore comprise one or more additional core modules each associated with one or more additional ORs, such as a second core module which may be associated with a second OR. The one or more additional core modules may be located remotely from the core module. That is, the ORs may be located in the same hospital environment, but are located sufficiently remotely that they are not physically connected through hardware. Instead, each core module in each OR connects to the same server, for example via the network.

Optionally, the system comprises a display, and the decoder is configured to provide the video stream to the display.

### BRIEF DESCRIPTION OF THE DRAWINGS

The invention will be described with reference to the following figures, in which:
Figure 1 is a schematic representation of an encoder according to aspects of the disclosure;
Figure 2 is an alternative schematic representation of an encoder according to aspects of the disclosure;
Figure 3A is a schematic representation of a video decoding unit of an encoder;
Figure 3B is a schematic representation of a video streaming unit of an encoder;
Figure 3C is a schematic representation of a snapshot frames unit of an encoder;
Figure 3D is a schematic representation of a video recording unit of an encoder;
Figure 3E is a schematic representation of a source frame unit of an encoder;
Figure 3F is a schematic representation of a live stream encryption unit of an encoder;
Figure 3G is a schematic representation of an ethernet subsystem unit of an encoder;
Figure 4 is a schematic representation of a decoder according to aspects of the disclosure;
Figure 5 is an alternative schematic representation of a decoder according to aspects of the disclosure;
Figure 6A is a schematic representation of an ethernet subsystem unit of a decoder;
Figure 6B is a schematic representation of a live stream decryption unit of a decoder;
Figure 6C is a schematic representation of a video streaming unit of a decoder;
Figure 6D is a schematic representation of a video encoding unit of a decoder;
Figure 7 is a method of encoding an operating room video feed according to aspects of the disclosure;
Figure 8 is another method of encoding an operating room video feed according to aspects of the disclosure;
Figure 9 is a schematic representation of an encoder according to aspects of the disclosure;
Figure 10 is a schematic representation of an operating room according to embodiments of the prior art;
Figure 11 is a schematic representation of a plurality of operating rooms on a hospital network according to embodiments of the prior art;
Figure 12 is a schematic representation of a system for obtaining medical data in an operating room according to aspects of the disclosure;
Figure 13 is a block diagram of a system for obtaining medical data in an operating room according to aspects of the disclosure;
Figure 14 is a schematic representation of a system for obtaining medical data in an operating room according to aspects of the disclosure; and
Figure 15 is a schematic representation of a system for obtaining medical data in a plurality of operating rooms according to aspects of the disclosure.

Like features are denoted by like reference numerals.

### DETAILED DESCRIPTION

### Encoder

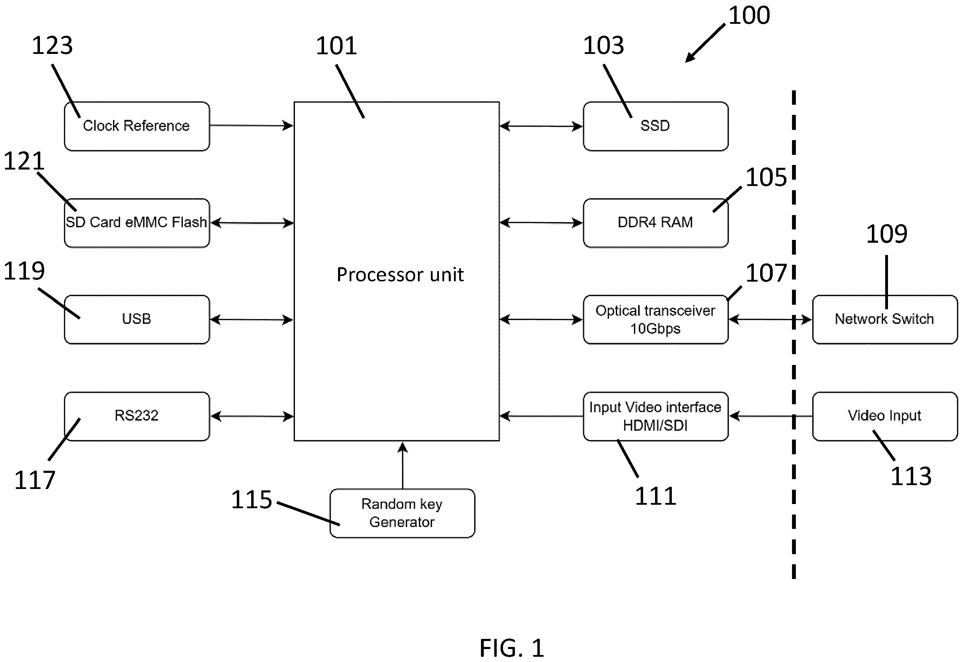

Figure 1 illustrates a schematic representation of an encoder 100 configured to perform aspects of the methods described herein. Encoder 100 comprises a processor unit 101 for processing the signals received by encoder 100. In particular, processor unit 101 is configured to perform various processes on received video streams, such as encoding of the video streams, and to enable network connectivity. Processor unit 101 may run on a variety of architectures and may comprise a number of sub-components or distinct microprocessors or processor sub-units within processor unit 101, for example, in the form of a multiprocessor system on a chip (MPSoC). In particular, processor unit 101 may comprise dedicated hardware for video encoding. A clock reference 123 is provided to processor unit 101 to provide a stable reference signal for timing.

Connected to the processor unit 101 may be one or more memory units, such as a solid state drive (SSD) unit 103 and a random access memory (RAM) unit 105. These memory units 103, 105 may provide storage for video during encoding or after it has been encoded by encoder 100. The SSD unit 103 is also used to save received video data, as discussed further below. Various other ports port and/or components may be provided for enabling connections to the encoder 100, such as SD card and/or eMMC 121, USB port 119, and RS-232 port 117. The firmware and operating system of the encoder 100 may be stored on SD card and/or eMMC flash memory 421 and at power up loaded by the processor unit 101.

A primary task of encoder 100 is to connect to video input sources 113 (e.g., from cameras, endoscopes etc.) and process the video to generate network compatible video feeds (e.g., internet protocol (IP) compatible feeds) to be transmitted on a network (e.g., an ethernet network capable of a bandwidth of 10Gbps). To this effect, encoder 100 is connectable to a network, such as the internet or a local area network (LAN) of a healthcare facility. This network connectivity functionality is provided by optical transceiver 107 which is connectable to a network switch 109, external from the encoder 100. Encoder 100 is also configured to receive input video, which it may then process and encode. Video input is received at input video interface 111 from a video input device 113. Preferably, input video interface 111 comprises a high-definition multimedia interface (HDMI) video input interface or four 3G-serial digital interfaces (3G-SDI), one of them able to manage 12G-SDI video signals.

Regardless of the input video interface 111 configuration used, encoder 100 is configured to simultaneously generate two video feeds from the video input: a first video feed that is a live compressed feed preferably having ultra low latency and/or visually lossless compression; and a second video feed that is a lossy low-bandwidth compressed feed having higher latency, and using lossy compression. Preferably, both the first and second video feeds are encoded into video over IP (VoIP) feeds.

The live compressed feed (i.e., the first feed) is the result of the input video processed by processor unit 101 with a light compressor algorithm that generates a data stream with minimum delay ready to be sent to a network.

The lossy low-bandwidth compressed feed (i.e., the second feed) may be a Dynamic Adaptive Streaming over Hypertext transfer protocol (DASH) compatible video feed generated by an standard video compressor (i.e. H.264, HEVC, AV1). The second feed has a lower bandwidth compared to the first, live compressed feed and an higher delay. This second feed enables the device to save the input video temporarily in memory, such as the SSD unit 103, before transferring the data to the central server over a network.

To ensure cybersecurity, the first, live compressed feed is encrypted with a random key that changes periodically. This random key can be provided by random key generator 115. Every other communication (including commands and the second, lossy low-bandwidth compressed feed) between the encoder 100 and the central server is preferably encrypted too, again utilising random key generator 115.

Upon request, the processor unit 101 of encoder 100 is also preferably capable of creating snapshots of the incoming video input. Encoder 100 also preferably has external visual components (not illustrated), such as one or more light emitting diodes (LEDs) to let a user know information about a power status (e.g., on, off, etc.), data transfer, input signal lock, SSD usage and if the operating system is running properly.

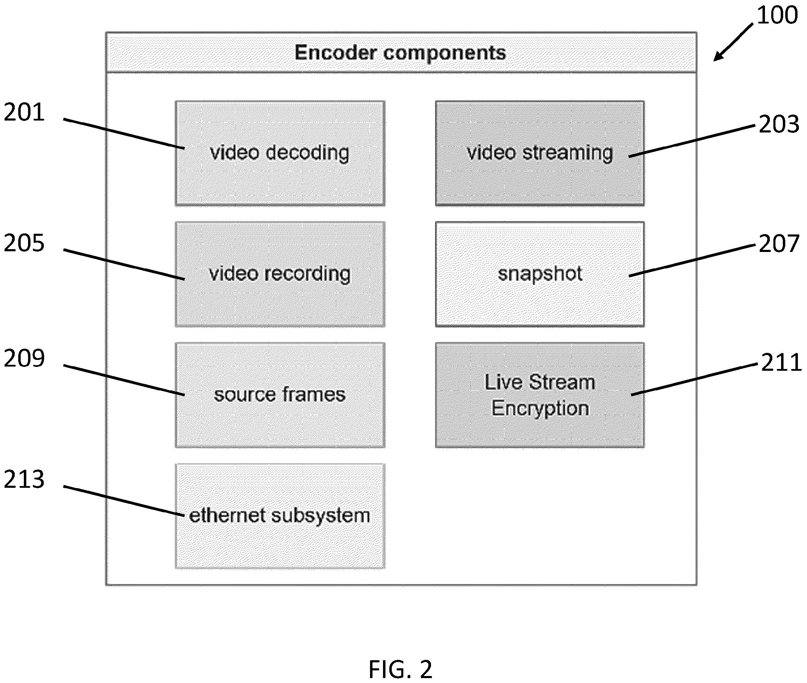

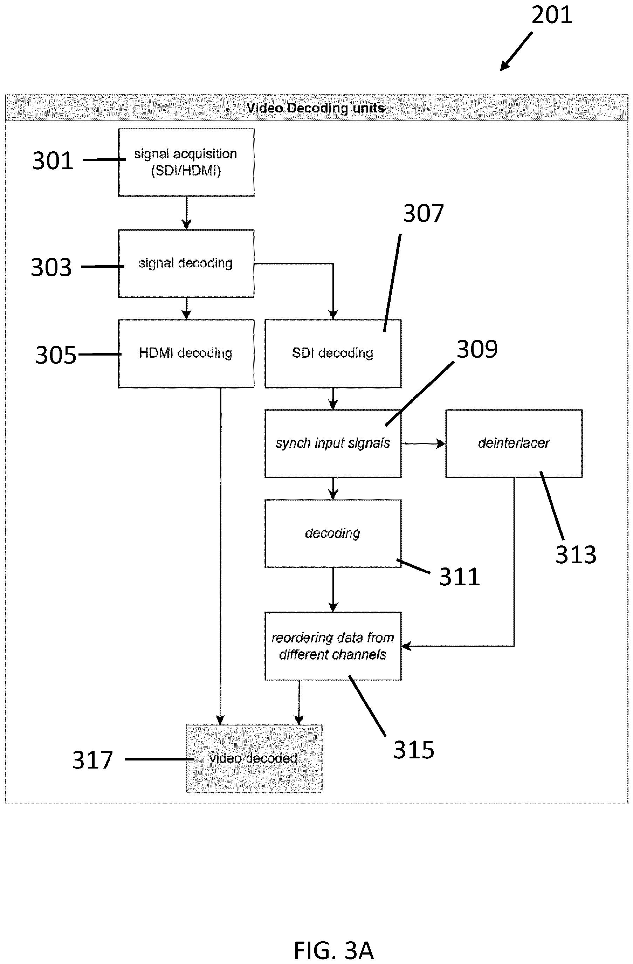

Figure 2 illustrates another schematic representation of encoder 100. This time, encoder 100 is represented as a number of functional blocks. Such functional blocks can be implemented within encoder 100 as some combination of hardware, software, and/or firmware. Encoder 100 comprises a video decoding block 201, a video streaming block 203, a video recording block 205, a snapshot block 207, a source frames block 209, a live stream encryption block 211, and an ethernet subsystem block 213. Figures 3A to 3G provide further detail about each of the functional blocks.

Figure 3A illustrates a functional representation of video decoding block 201, illustrating the steps performed by video decoding block 201. Initially, video decoding block 201 obtains a signal at signal acquisition block 301. This signal may be HDMI or SDI, for example, as discussed above. The acquired signal is then decoded at signal decoding block 303. The decoding will vary depending upon the type of video signal. For example, it will vary between HDMI and SDI video input.

For HDMI video, the signal is decoded at HDMI decoding block 305 and is then output as a decoded video at block 317. For SDI video, the signal is decoded at SDI decoding block 307. If four 3G-SDI input signals are received, they are synchronised by synchronisation block 309 and reordered into one 12G-SDI output signal. The synchronised signals may then be decoded by decoding block 311 or deinterlaced by deinterlacing block 313, before the signals from the four channels are then reordered into the correct playback order by reordering block 315, to output the decoded video at block 317.

Generally, an encoder will be configured to receive a video input such as through HDMI, DisplayPort (in which case video will be processed in substantially the same was as video received through and HDMI port)or SDI video input, which are mutually exclusive, so in encoder 100 there may generally only be one firmware depending upon the video signal input. In some cases, both paths may be provided so that an encoder 100 may be able to receive input in either HDMI or SDI form.

As the input video is decoded by video decoding block 201, the data is processed in parallel other functional components of the encoder 100.

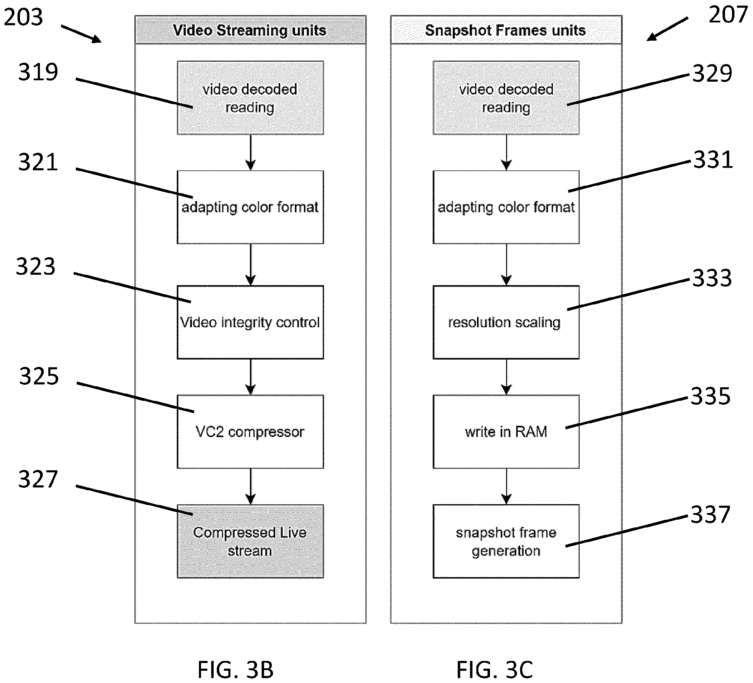

Figure 3B illustrates a representation of the functions performed by the video streaming block 203. Video streaming block 203 receives the decoded video output from video decoder block 201 at block 319. The colour format of the decoded video feed is adapted at block 321 before integrity control is performed at block 323. Subsequently, the video feed is then compressed into the first, live compressed video feed by video compressor block 325. This compression is a visually lossless light compression introducing no (or practically no) latency, to give an output live stream at block 327.

Figure 3C illustrates a representation of the functions performed by the snapshot frame block 207. As with video streaming block 203, snapshot frame block 207 receives the decoded video output from the video decoder block 201. This is received in the snapshot frame block 207 at block 329. Again, similarly to the video streaming block 203, the colour format of the decoded video feed is adapted at block 331 before integrity control is performed at block 333. From here, the decoded video feed is written into the RAM memory at block 335, enabling short term storage of the video feed so that snapshots (i.e., video frames) can be obtained at block 337, for example, for long term storage or distribution.

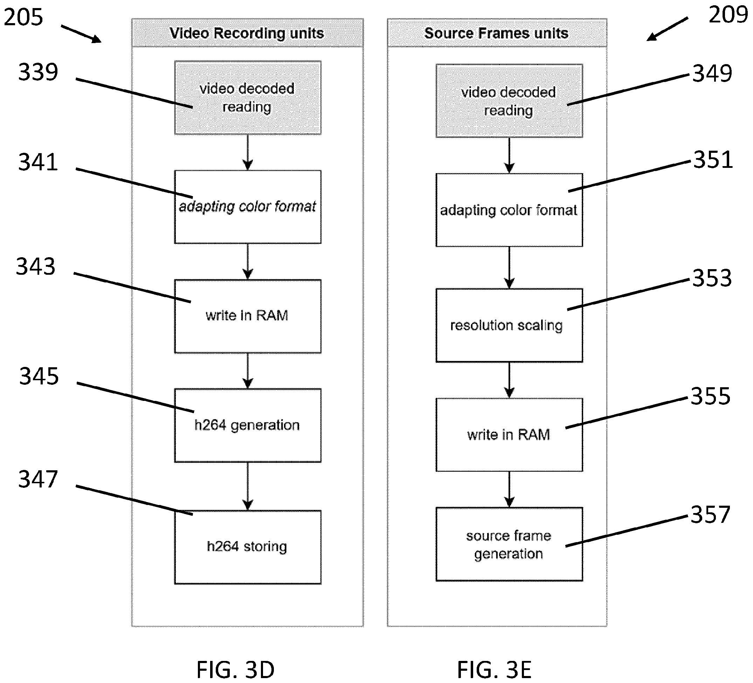

Figure 3D illustrates the functionality of the video recording block 205 of the encoder 100. The decoded video feed is received, at block 339, from the video decoding block 201, and its colour format is adapted at block 341. It is subsequently written into RAM at block 343 as a buffer, from where it can then be accessed by encoder block 345. In the illustrated example, encoder block 345 is an H.264 encoder, though other video codecs such as HEVC and AV1 could also be used. Generally, the encoding performed by encoder block 345 of the video recording unit 205 is a higher compression than the compression performed by compressor block 325 of the video streaming block 203. Once encoded into the compressed format by encoder block 345, the compressed video feed can be stored at block 347. This compressed video feed is the second video feed and can have a lower bit rate and higher latency as it is not used for live viewing. As well as for storage, this second video feed can also be streamed over external networks (such as the internet) in asynchronous feeds, and used for other purposes that don't require a live (i.e., very low latency) feed.

Figure 3E illustrates a representation of the functions performed by the source frame block 209. These functions are very similar to those performed by the snapshot frame block 207. Source frame block 209 receives the decoded video output from the video decoder block 201 at block 349. The colour format of the decoded video feed is adapted at block 351 before integrity control is performed at block 353. Next, the decoded video feed is written into the RAM memory at block 355, enabling short term storage of the video feed so that source frames can be obtained at block 357, which may be used, for example to generate a preview of the video stream or other functions that do not need to use the full resolution of the video.

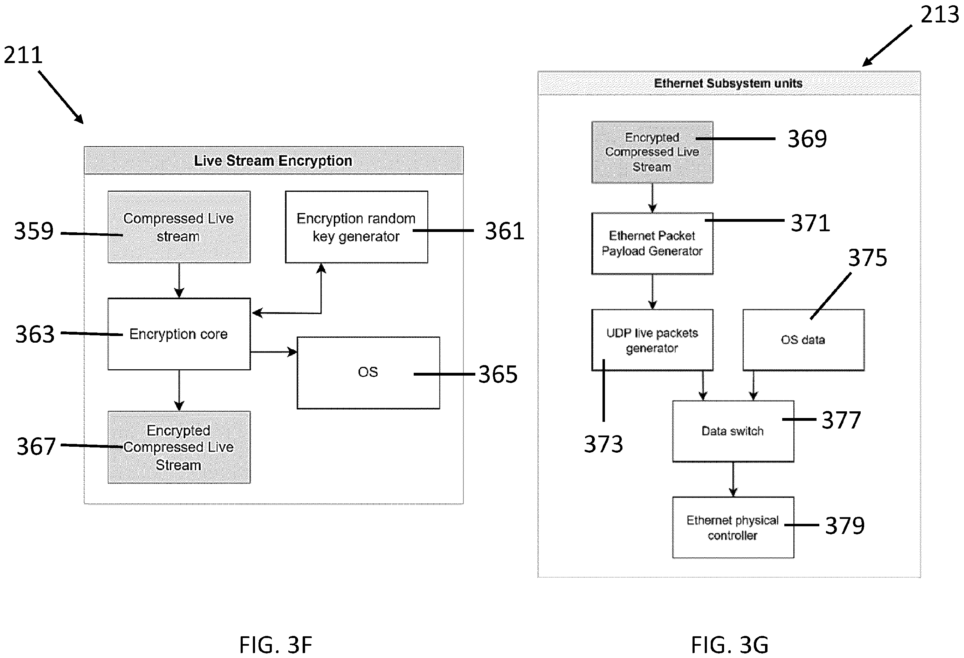

Figure 3F shows a representation of the live stream encryption block 211. The live stream encryption block 211 takes the live stream, that is, the lightly compressed first video feed, at block 359 and encrypts it with the encryption core 363. This utilises a random key, generated and received from random key generator 361. The encrypted and compressed live stream is then output at block 367. OS block 365 communicates the encryption keys over a safe (encrypted) network channel, e.g., to a server.

Figure 3G illustrates the functionality of the ethernet subsystem block 213. The ethernet subsystem block 213 received, from the live stream encryption block 211, the encrypted live (first) video feed at block 369. This is passed to the ethernet packet payload generator 731 and then onto the user datagram protocol (UDP) packet generator 373 for generating the data packets for distribution on a network. A data switch 377 then receives the UDP live feed packets from the UDP packet generator 373, along with encryption keys from block 375, which works with ethernet physical controller 379 to communicate the encrypted compressed live stream over a network, in this case an ethernet network. This network allows live viewing of the first video feed by connected devices, but must be a network capable (e.g., having sufficient bandwidth) of sharing the first video feed with low latency.

### Decoder

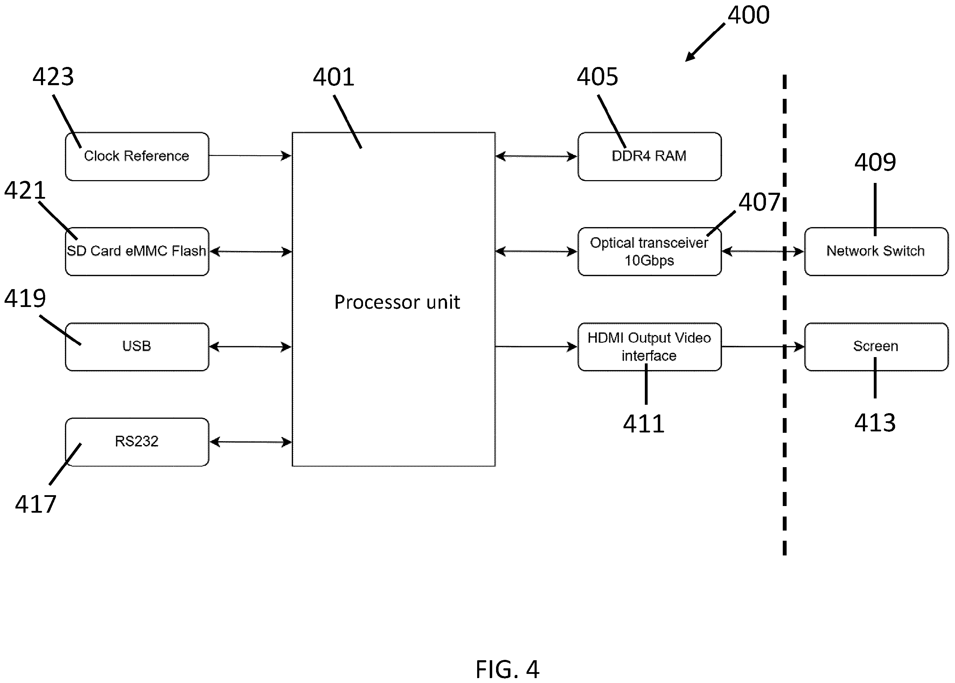

Figure 4 illustrates a schematic representation of a decoder 400 configured to perform aspects of the methods described herein. Many aspects of the decoder 400 are similar or the same as aspects of encoder 100, described with reference to Figure 1.

Like encoder 100, decoder 400 comprises a processor unit 401 for processing the signals received by decoder 400. In particular, processor unit 401 is configured to perform various processes on received video streams, such as decoding of the video streams received from a network for output and viewing. Processor unit 401 may run on a variety of architectures and may comprise a number of sub-components or distinct microprocessors or processor sub-units within processor unit 401, for example, in the form of a MPSoC. In particular, processor unit 401 may comprise dedicated hardware for video decoding. A clock reference 423 is provided to processor unit 401 to provide a stable reference signal for timing.

Connected to the processor unit 401 may be one or more memory units, such as a RAM unit 405. Memory units 405 may provide storage a video feed during decoding of the video feed. Various other ports may be provided for enabling connections to the decoder 400, such as SD card and/or eMMC port 421, USB port 419, and RS-232 port 417. The firmware and operating system of the decoder 400 may be stored via SD card and/or eMMC flash memory 421.

Decoder 400 receives and processes data received from a network. For example, data is received at transceiver 407 from the network via a connected network switch 409, which may be an ethernet switch, external to the decoder 400. The data received from network switch 409 may be commands sent over the network or a video live compressed stream (e.g., the first video feed) coming from an encoder 100 connected to the same network switch 409 (i.e., network switch 109 and network switch 409 may be the same physical switch if encoder 100 and decoder 400 are connected to the same network).

In the case of a compressed video, the decoder 400 decrypts (if necessary) and decompresses the video stream in the processor unit 401. The output decrypted and decompressed video feed is sent to the HDMI output interface 411 ready to be displayed by any device with an HDMI input port, such as a screen 413. Preferably, the decoder 400 determines the best display resolution to use for a connected screen 413 and adapts the video output accordingly.

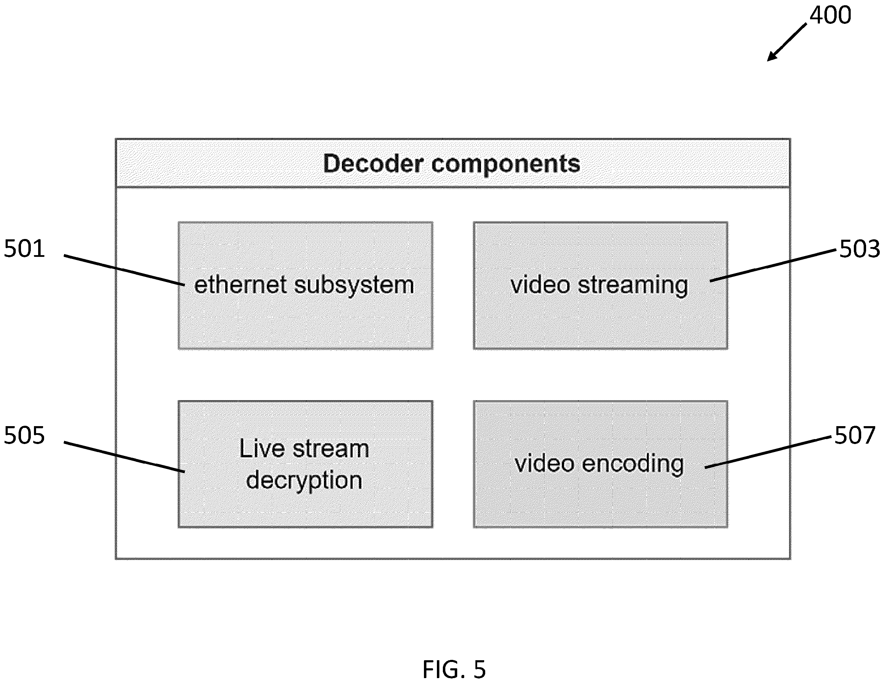

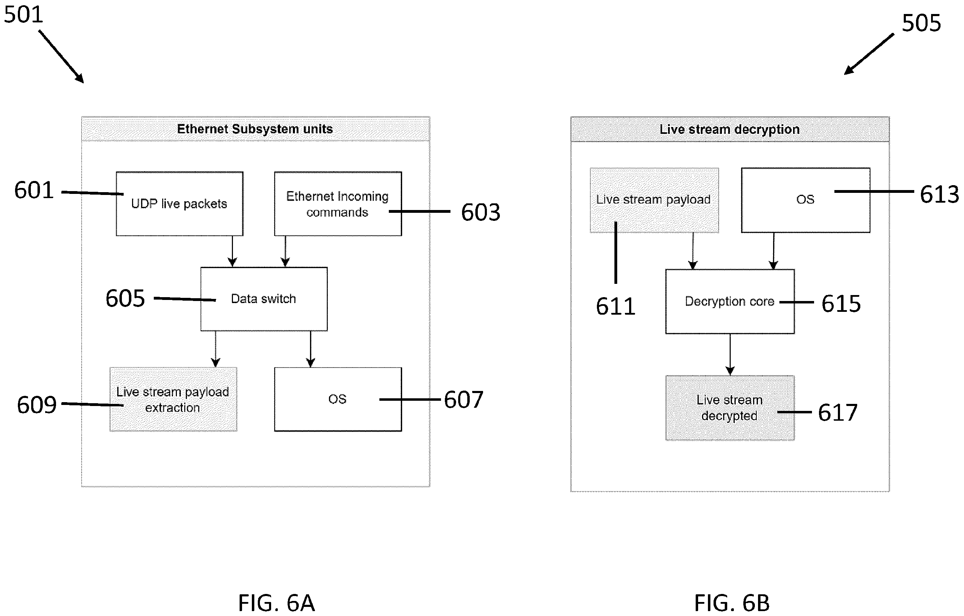

Figure 5 illustrates another schematic representation of decoder 400. This time, decoder 400 is represented as a number of functional blocks. Such functional blocks can be implemented within decoder 400 as some combination of hardware, software, and/or firmware. Decoder 400 comprises an ethernet subsystem block 501, a video streaming block 503, a live stream decryption block 505, and a video encoding block 507. Figures 6A to 6D provide further detail about each of the functional blocks.

Figure 6A illustrates a functional representation of the ethernet subsystem block 501. Figure 6A illustrates the functional processes performed by the ethernet subsystem block 501. The ethernet subsystem block 501 receives data from the network. This may be in the form of UDP data packets of the first, live video feed (e.g., sent over then network from encoder 100) at block 601, or incoming commands at block 603. This incoming data is passed to data switch 605 which can then either pass the data to the processor (e.g., if the data comprises incoming commands) at block 607 or output the live feed data for decryption at block 609.

Figure 6B shows a functional diagram of live stream decryption block 505. The live stream decryption block 505 receives, at block 611, the live feed output from block 609 of the ethernet subsystems block 501. The live stream decryption block 505 also receives decryption instructions from processor at block 613, and the live feed is then decrypted in the decryption core 615. The live feed is then output from the live stream decryption block 505 as a decrypted feed at block 617.

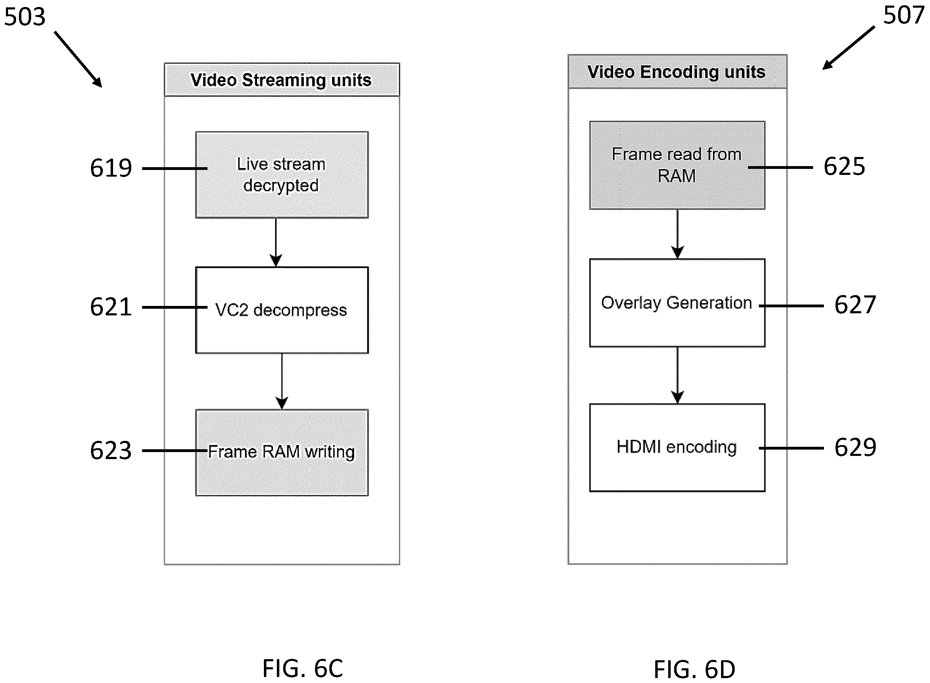

Figure 6C illustrates the functionality of video streaming unit 503. Video streaming unit 503 takes, at block 619, the decrypted live feed that has been decrypted by the live stream decryption block 505 and output at block 617. It then un-compresses it (e.g., the reverse operation that block 325 in the encoder performs), at block 621. The un-compressed live feed is then buffered in RAM at block 623.

Figure 6D illustrates a functional representation of the video encoding block 507, which utilises the buffered live feed (prepared at block 623 of video streaming block 503). This is received at block 625 of the video encoding block 507. From here, the buffered stream goes through overlay generation block 627 to generate any images or symbols to be overlayed on video before being encoded for HDMI output at block 629. This encoded feed can then be output to a display, such as screen 413. It will be appreciated that whilst HDMI encoding is used in the illustrated example, other types of encoding and output may be used depending, for example, upon the type of display the output is to be displayed on.

### Method of Operating Encoder

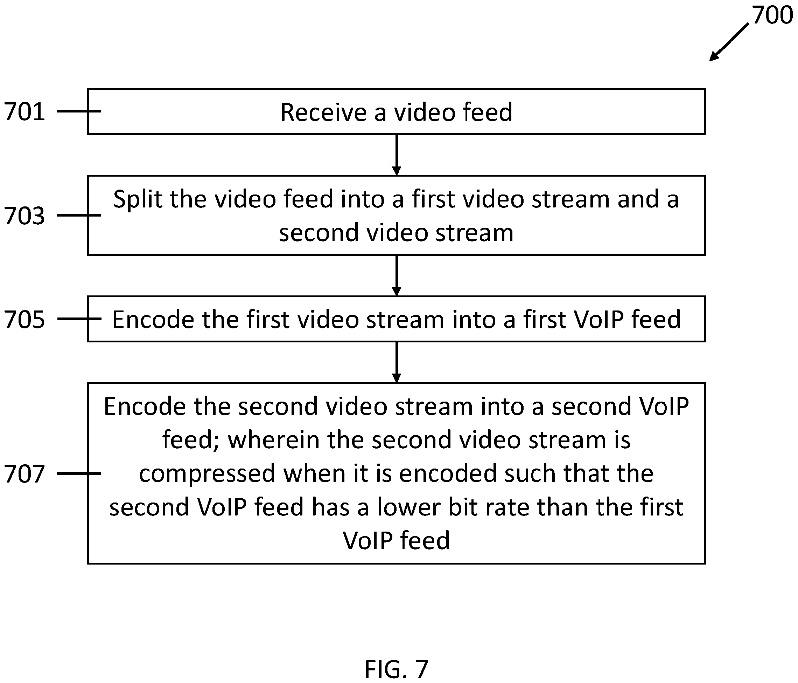

A method of encoding an operating room video feed into a VoIP steam will now be described with respect to Figure 7. In particular, parts or all of the method may be performed by the encoders described herein, such as by encoder 100.

Method 700 begins at step 701 wherein a video feed is received. These may be received from one or more cameras in an operating room, such as a video of a surgical procedure. For example, encoder 100 may receive a video feed at Input Video interface 111. The video may in particular be a HD video feed.

At step 703, the video feed is split into a first video stream and a second video stream and then the first video stream and second video stream are encoded into VoIP feeds. Splitting the video feed into a first video stream and a second video stream enables different encoding to be performed on the two video streams. The two video streams can, therefore, then be optimised for different uses, such as streaming and recording.

Specifically, at step 705, the first video stream is encoded into a first VoIP feed and at step 707, the second video stream is encoded into a second VoIP feed. The encoding of the first video stream and the second video stream into the first VoIP feed and the second VoIP feed, at steps 705 and 707 respectively, differ in the compression used. The second video stream is compressed when it is encoded such that the second VoIP feed has a lower bit rate than the first VoIP feed.

The first video stream may be uncompressed when it is encoded into the first VoIP feed at step 705, or, more preferably, encoded using visually lossless compression. The second video stream may be compressed, when it is encoded into the second VoIP feed at step 707, using High Efficiency Video Coding (HEVC), also known as H.265; Advanced Video Coding (AVC), also known as H.264; or AOMedia Video 1 (AV1) compression. Encoding the first video stream into a visually lossless first VoIP feed provides a low latency (i.e., real time), visually lossless feed that is suitable for local use within a local environment of the encoder (especially a wired network environment), such as within an operating room or healthcare environment. On the other hand, the more heavy compression applied to the second video stream to provide the second VoIP feed gives a much lower bandwidth feed suitable for streaming over a network, in particular an external network such as the internet or a wireless network, as well as other applications that do not require real time video, such as archiving. In one example, the first VoIP feed may have a bit rate of between 5 and 6 Gbps and the second VoIP feed may have a bit rate of less than 0.2 Gbps.

In some cases, the encoding of the first video stream and/or the encoding of the second video stream may be based upon one or more properties of the video feed. In this case, the method may further comprise determining at least one property of the video feed. For example, the determined property may be a format of the received video feed, a resolution, bit rate, and the like. In one example, different codecs may be used to encode the first video stream and/or the second video stream depending upon the bit rate of the video feed so as to ensure that the first VoIP feed and/or the second VoIP feed have the desired bit rates. In another example, encoding the first video stream and/or encoding the second video stream may comprise scaling the first video stream and/or scaling the second video stream based on the resolution of the video feed and/or based on a resolution of a display to which the first VoIP feed or the second VoIP feed is to be output to.

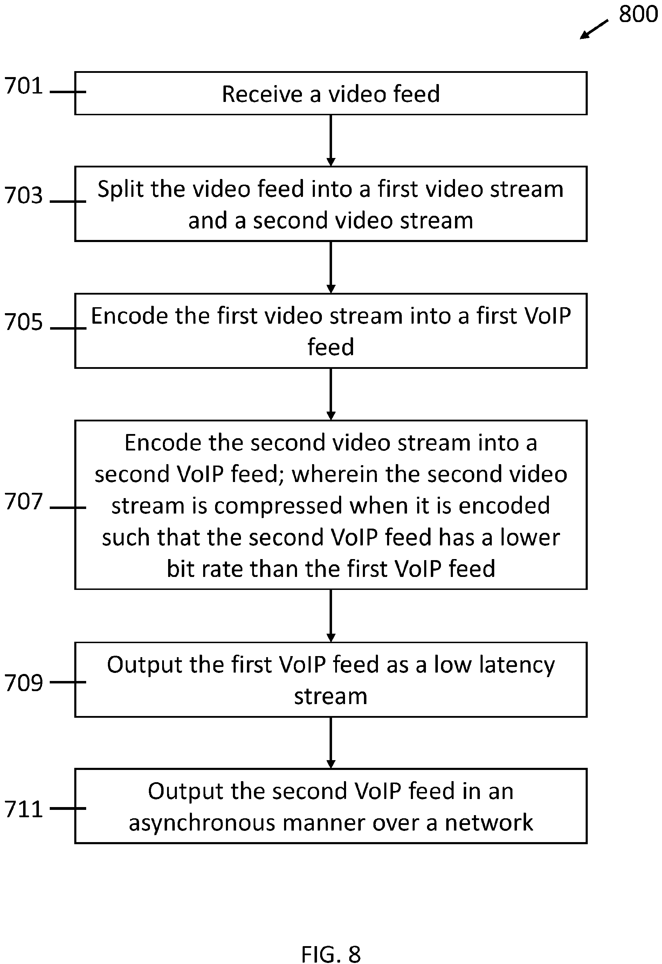

Methods according to the present disclosure may also comprise outputting the first VoIP feed and the second VoIP feed. Figure 8 illustrates such a method 800. Method 800 comprises all of the steps 701 to 707 of method 700, as described above. Therefore, these steps will not be described in detail again.

However, after the first video stream is encoded into the first VoIP feed at step 705 and the second video stream is encoded into the second video feed at step 707, method 800 comprises output steps 709 and 711. At step 709, the first VoIP feed is output as a low latency stream. As noted above, this makes the first VoIP feed suitable for real time applications and in particular local applications, such as viewing a feed from a camera within an operating room in which the camera is recording.

At step 711, the second VoIP feed is output as an asynchronous stream. This may be done by first dividing the second VoIP feed into a plurality of fragments and then sending the plurality of fragments in an asynchronous manner over a network. Before outputting the plurality of fragments in an asynchronous manner, one or more of the plurality of fragments may be buffered.

Outputting the second VoIP feed in an asynchronous manner means that, while not suited for low latency, real time uses, the burden on the network over which the asynchronous, second VoIP feed is transmitted is greatly reduced. Because there is no requirement for the fragments of the second VoIP feed to arrive at their destination in a particular order or at a particular time, each fragment can be sent via different paths through the network that may take different times, but that help distribute the network load more evenly and improve overall network performance.

Once the fragments are received at their destination, they can be stored either in their fragmented state or in a recombined state. That is, the method 800 can further comprise a step of receiving, at a server, the plurality of fragments and then storing, on the server, the plurality of fragments. If stored in the fragmented state, they should be stored with the information that will enable the second VoIP feed to be reconstructed from the fragments. Alternatively, the server can reconstruct the second VoIP feed by combining the plurality of fragments into an ordered playlist or by combining the plurality of fragments into a single file recreating the second VoIP feed. In this manner, the second VoIP feed can be utilised for non-real time uses such as streaming over the internet and long term storage or archiving.

The methods described herein may be performed on a single computing device (on one or more processors), or across multiple computing devices in a distributed system. Such a distributed system may be connected by a local area network or a wide area network. The methods may also take advantage of cloud computing services to perform one or more steps of the method.

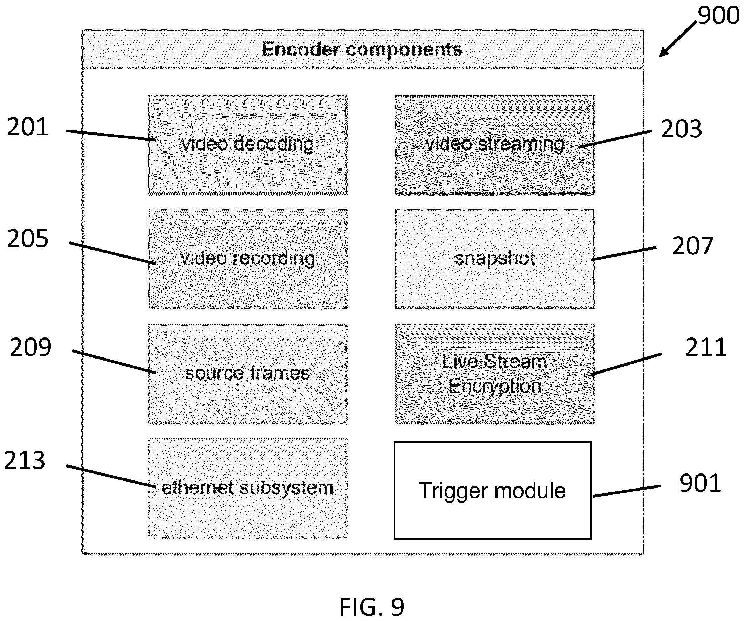

Figure 9 illustrates an encoder 900 according to embodiments of the present disclosure. The encoder 900 of Figure 9 is largely similar to the encoder described in relation to Figure 2. However, significantly, the encoder 900 also comprises a trigger module 901. The trigger module is configured to, in response to a trigger command, trigger the recording module to begin recording a video stream or video feed of medical data.

In this example, the trigger command comprises the receipt, by the encoder 900, of a video stream. The encoder 900 is part of a system also comprising a video stream source, and the encoder 900 is in communication with the video stream source. When the encoder 900 receives a video stream from the video stream source, the encoder automatically begins to record the video stream.

In this example, the encoder 900, and in particular the recording block or recording module 205, is configured to buffer at least some of the video stream to the local storage. This local buffering is also performed automatically as soon as the encoder 900 receives the video stream.

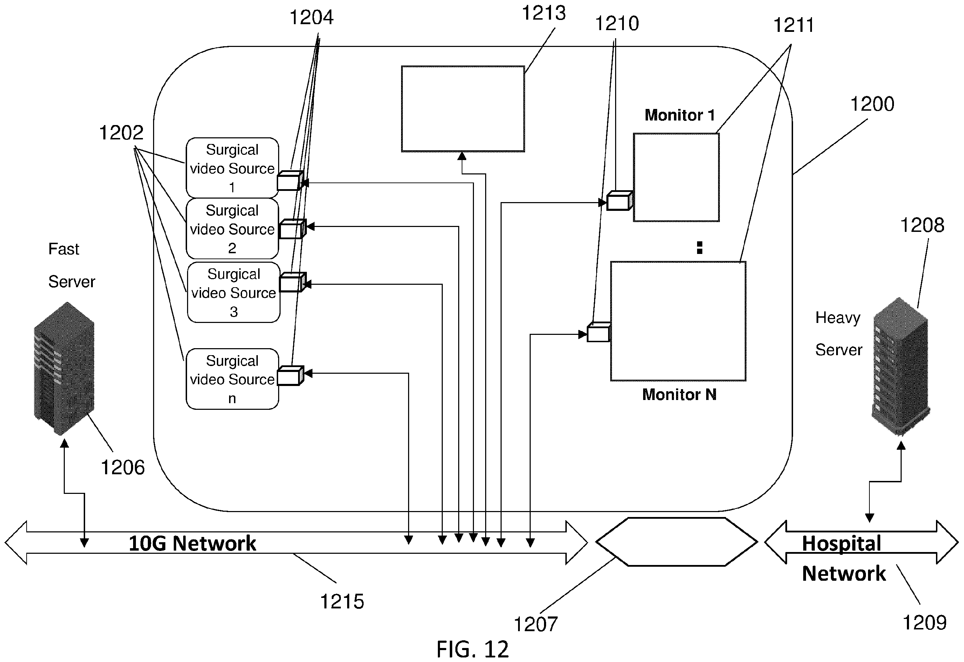

The encoder 900 is in communication with a server architecture (not shown) which comprises a media asset manager. When it is determined that at least some of the buffered video stream stored locally at the encoder is of interest, the video stream determined as being of interest is transmitted or provided to the media asset manager of the server architecture via a network. In this example, a portion of a video stream relating to medical data is determined as being of interest in response to a user interaction. A user provides an input, either through a control interface located within an OR, or remotely through the centralized system provided by the server architecture (for example, through the media asset manager), indicating that a particular portion of the video stream is of interest. The video stream of interest is then provided to the media asset manager. The larger network is illustrated with respect to Figures 12 to 15 described below.

Figures 12 to 14 illustrate the system of a single OR in connection with the wider server architecture.

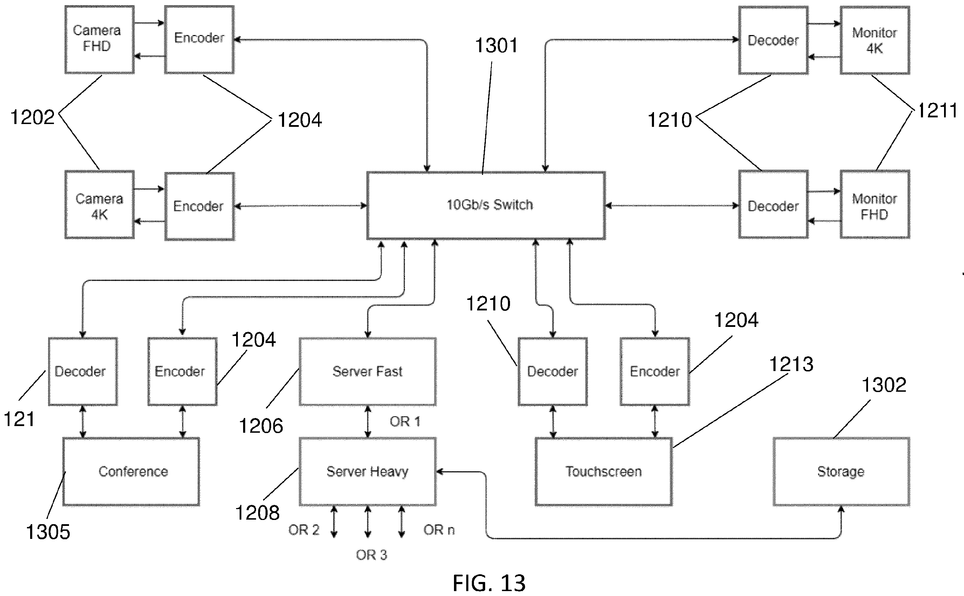

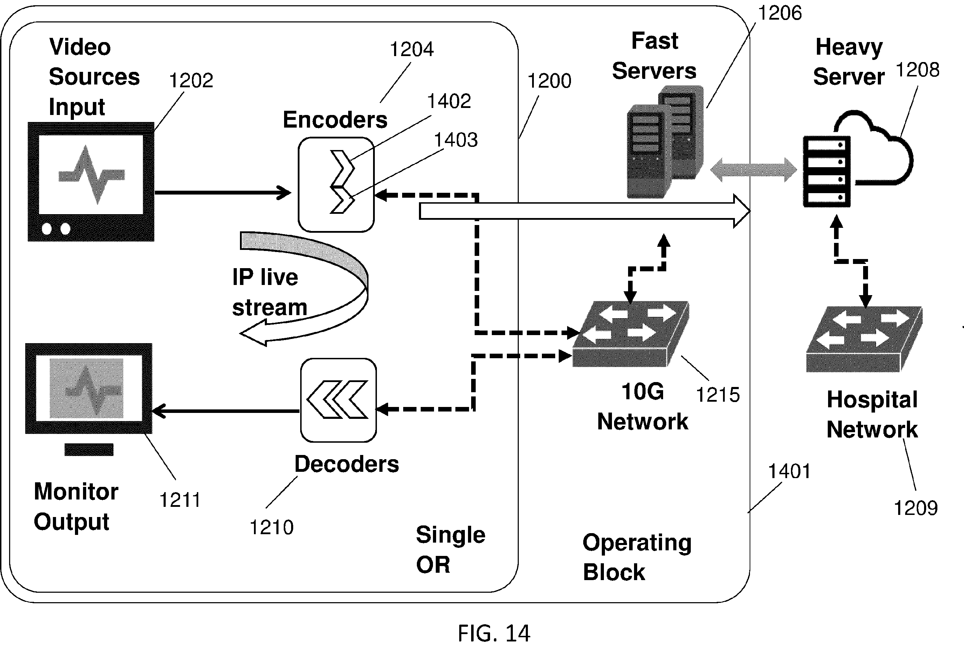

Figure 12 illustrates an OR 1200 incorporating the distributed intelligence system described herein. The system is a system for obtaining medical data in one or more operating rooms. Figure 13 illustrates a block diagram of components of a corresponding OR. Figure 14 similarly illustrates a single OR implementing the distributed intelligence system, and Figures 12 to 14 will now be described in conjunction.

The OR 1200 comprises a plurality of video stream sources 1202 including devices 1, 2, 3... n. In this example, the video stream sources 1202 include surgical cameras such as endoscopes and x-ray imaging cameras. Each video stream source 1202 is coupled to an encoder 1204. The encoders 1204 may incorporate the functionality of any encoder described herein. In this example, each encoder 1204 is configured to receive and record a video stream of medical data from its respective video stream source 1202.

Significantly, each of the encoders 1204 is coupled to a core module 1301 (illustrated in Figure 13) associated with the OR 1200. The core module 1301 provides, among other things, communication with a server architecture including a fast server 1206 ad a heavy server 1208. In this example, the core module 1301 comprises a 10Gbps switch and is configured to connect to the server architecture through a network infrastructure. In particular, in this example, the fast server 1206 is located in relatively close proximity to the OR 1200, for example at an OR block including a plurality of ORs 1200, and is in connection with the core module via an ethernet network 1214 capable of providing 10Gbps bandwidth.

The fast server 1206 and the 10Gbps network additionally connects to a wider hospital network 1209 on which the heavy server 1208 operates, via a network trunk 1207. The fast server provides functionality to the operating room through the network infrastructure. In this example, the fast server 1206 provides videoconferencing 1305 functionality to the OR through the necessary software and processing including both video and audio management.

In this example, the heavy server 1208 is a virtualized server, and the heavy server 1208 provides services not necessarily being required in real-time, and may also provide the functionality related to other software including RTLS, a scheduling system, and a streaming platform. The heavy server 1208 comprises a media asset manager 1302 to which video streams are sent. That is, encoders 1204 receive and locally buffer video streams according to embodiments described herein. The media asset manager then receives a user input indicating that at least some of the video streams are of interest. Having received the indication, the video streams of interest are sent from the encoders to the heavy server 1208 via the core module and network infrastructure. Medical data, for example in the form of video streams, may then be accessed centrally through the hospital network on which the heavy server 1208 operates.

The OR 1200 also comprises a plurality of displays or monitors 1211 from monitor 1 to n. The monitors 1211 are each coupled to a respective decoder 1210, which are in turn in communication connection with the core module. The decoders 1210 may include the functionality of any of the embodiments described herein, but briefly receive and provide a video signal for display on corresponding monitors 1211.

Encoders 1204 and decoders 1210 are connected to respective video stream sources 1202 and monitors 1211 with any suitable connection as described herein, for example using HDMI interfaces or SDI interfaces.

The OR further comprises a control interface 1213. In this example, the control interface 1213 is coupled with the core module and comprises a touch screen interface. The control interface 1213 allows for control of the various devices located within the OR 1200. For example, the control interface 1213 may provide control of the encoders 1204, decoders 1210, video source devices 1202, and monitors 1211. The control interface 1213 is also used for videoconferencing functionality provided by the fast server 1206.

The core module comprises a plurality of ports facilitating connection between the plurality of encoders 1204, the plurality of decoders 1210, and the server architecture. Central processing is provided through the server architecture. In this example, the system comprises a cloud based computing system The application of a local fast server 1206 working in a small local cloud provides support for each individual OR 1200.

Figure 14 illustrates a single OR 1200 as described with respect to Figures 12 and 13, but particularly illustrates the operating block 1401 in which a plurality of ORs may be situated.

In addition, Figure 14 also particularly illustrates the incorporation of the simultaneous generation of two video feeds at the encoder including a first video feed 1402 that is a live compressed feed having ultra-low latency and/or visually lossless compression, and a second video feed 1403 that is a lossy low-bandwidth compressed feed having higher latency, and using lossy compression. This may be performed according to embodiments described herein.

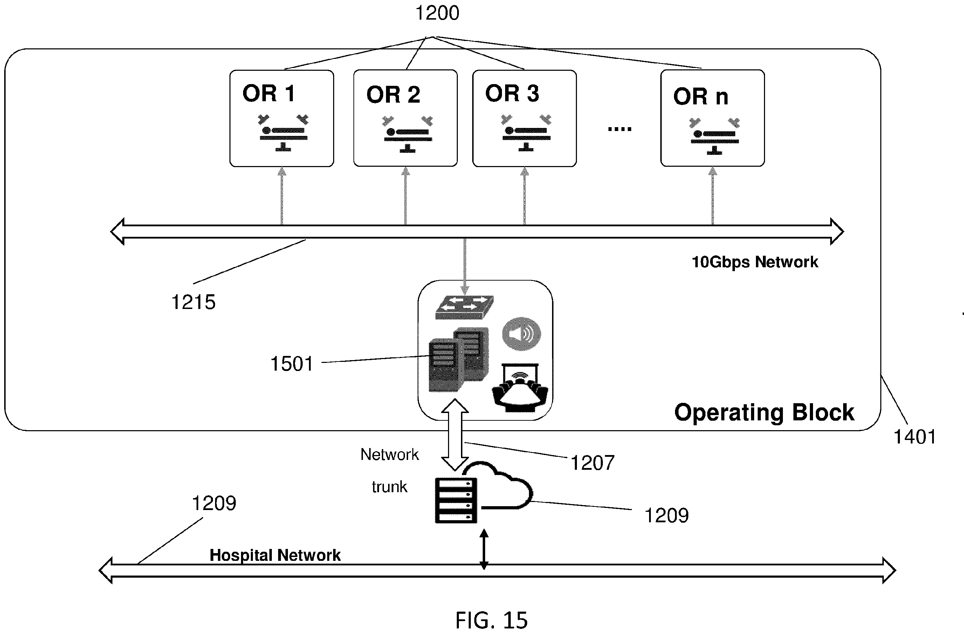

Figure 15 illustrates said operating block 1401 including a plurality of ORs 1200 including ORs 1 to n.

Each of the ORs 1200 includes arrangements as described, but could include any variation of devices, encoders, and decoders. Significantly, each of the ORs 1200 is in communication with the same server infrastructure, being in communication with the fast server 1206 via core modules located at each OR 1200, and ultimately in communication with the larger hospital network and heavy server 1208 via the network trunk.

Figure 15 illustrates the ease of expansion and scalability with the described distributed intelligence system. Figure 15 particularly illustrates a plurality of ORs 1200 in one operating block 1401, the plurality of ORs 1200 being in communication with a "rack" 1501. In this example, this rack 1501 includes the fast server 1206, the 10Gbps switches associated with each OR 1200, a videoconferencing unit for providing videoconferencing functionality, and an audio management unit.

The "intelligence" or processing functionality is provided by individual encoders and decoders, and devices located within the server architecture. While video streams may be buffered locally at each encoder coupled to each video stream source in each OR, the video streams are provided to, and stored at, a media asset manager at the heavy server. The video streams may then be processed and viewed by users via a centralized intelligence system. The system therefore provides distributed processing across a network covering a plurality of ORs 1200, but also provides centralized processing per OR 1200 due to the functionality of devices such as encoders and decoders. In order to expand the system to include additional ORs, a core module is installed in the OR and coupled to relevant devices, which is then in communication with the distributed system. This removes the need to installing an entire hardware/software "stack" in each OR.

## Claims

1. A method of encoding an operating room video feed into a video over internet protocol (VoIP) stream, the method comprising:
receiving a video feed;
splitting the video feed into a first video stream and a second video stream;
encoding the first video stream into a first VoIP feed;
encoding the second video stream into a second VoIP feed;
wherein the second video stream is compressed when it is encoded such that the second VoIP feed has a lower bit rate than the first VoIP feed.

2. The method of claim 1, wherein the second video stream is compressed using one of HEVC, H.264, and AV1 compression.

3. The method of claim 1 or 2, wherein the first video stream is compressed when it is encoded into the first VoIP feed.

4. The method of claim 3, wherein the first stream is encoded using a visually lossless compression.

5. The method of claim 3 or 4, wherein the first video stream is encoded such that the first VoIP feed has a bit rate of between 5 and 6 gigabits per second and/or wherein the second video stream is encoded such that the second VoIP feed has a bit rate of less than 0.2 gigabits per second.

6. The method of any preceding claim, wherein the method further comprises:
determining at least one property of the video feed;
wherein the encoding of the first video stream and/or the encoding of the second video stream is based upon the determined at least one property of the video feed.

7. The method of claim 6, wherein the at least one determined property of the video feed comprises a resolution of the video feed, and wherein the encoding of the first video stream and/or the encoding of the second video stream is based upon the resolution of the video feed.

8. The method of claim 7, wherein the encoding of the first video stream and/or the encoding of the second video stream comprises scaling the first video stream and/or scaling the second video stream based upon the resolution of the video feed.

9. The method of claim 8, wherein the method further comprises receiving a resolution of a display, and wherein scaling the first video stream and/or scaling the second video stream is further based upon the resolution of the display.

10. The method of any preceding claim, wherein the method further comprises:
outputting the first VoIP feed as a low latency stream; and
outputting the second VoIP feed as an asynchronous stream.

11. The method of claim 10, wherein outputting the second VoIP feed as an asynchronous stream comprises:
dividing the second VoIP feed into a plurality of fragments; and
sending the plurality of fragments in an asynchronous manner over a network.

12. The method of claim 11, wherein prior to outputting the plurality of fragments in an asynchronous manner, one or more of the plurality of fragments are buffered.

13. The method of claim 11 or 12, wherein the plurality of fragments are output from an encoder or a switching device, and wherein the method further comprises:
receiving, at a server, the plurality of fragments; and
storing the plurality of fragments on the server;
wherein optionally the method further comprises combining, by the server, the plurality of fragments into an ordered playlist of the fragments or to recreate the second VoIP feed.

14. A video encoder configured to perform the method of any of claims 1 to 12; or a system comprising a video encoder and a server configured to perform the method of claim 13.

15. A non-transitory computer readable memory having stored thereon instructions that, when executed by a video encoder according to claim 14, cause the video encoder to perform the method of any of claims 1 to 12; or having stored thereon instructions that, when executed on a system according to claim 14, cause the system to perform the method of claim 13.
